# EUROPÄISCHE PATENTSCHRIFT

(11) **EP 2 803 368 B1**
(45) Veröffentlichungstag und Bekanntmachung des Hinweises auf die Patenterteilung: **16.11.2016**
(21) Anmeldenummer: 14001716.1
(22) Anmeldetag: 15.05.2014
(51) Int. Cl.: A61L 2/08, B65B 55/08

(54) **Vorrichtung und Verfahren zum Sterilisieren von Behältnissen mit Abschirmeinrichtung gegen Röntgenstrahlen**
Device and method for sterilising containers with x-ray shielding device
Dispositif et procédé destinés à la stérilisation de récipients dotés de dispositif de blindage contre les rayons X

(30) Priorität: 15.05.2013 DE 102013104998
(43) Veröffentlichungstag der Anmeldung: 19.11.2014
(73) Patentinhaber: Krones AG, 93073 Neutraubling (DE)
(72) Erfinder: Scheuren, Hans, 93073 Neutraubling (DE); Knott, Josef, 93073 Neutraubling (DE); Neubauer, Michael, 93073 Neutraubling (DE)
(74) Vertreter: Bittner, Bernhard

(56) Entgegenhaltungen:
- EP-A1- 2 371 397
- EP-A1- 2 724 731
- EP-A1- 2 769 740
- EP-A1- 2 769 922
- EP-A2- 2 594 493
- WO-A1-2013/092735

## Beschreibung

Die vorliegende Erfindung bezieht sich auf eine Vorrichtung und ein Verfahren zum Sterilisieren von Behältnissen. Aus dem Stand der Technik sind diverse derartiger Verfahren bekannt und werden beispielsweise eingesetzt, um Kunststoffbehältnisse und insbesondere Kunststoffvorformlinge vor ihrer weiteren Verarbeitung zu sterilisieren. Dabei sind seit langem Sterilisationsverfahren bekannt, bei denen die Kunststoffbehältnisse einem Sterilisationsmittel, wie beispielsweise Wasserstoffperoxid oder Peressigsäure, ausgesetzt werden. In jüngerer Zeit ist man jedoch auch dazu übergegangen, zur Sterilisation elektromagnetische Strahlung, wie beispielsweise Elektronenstrahlen, ultraviolette oder Röntgenstrahlen einzusetzen. Der Einsatz derartiger Strahlen ist auf der einen Seite ressourcenschonender, da kein zusätzliches Sterilisationsmittel eingesetzt werden muss, der Nachsteil besteht jedoch darin, dass oftmals gefährliche Strahlung, wie beispielsweise ionisierende Strahlung, insbesondere Röntgenstrahlung, entsteht.

EP 2 371397 offenbart die im Oberbegriff enthaltenen Merkmale.

Beispielhafte Vorrichtungen zum Sterilisieren von Behältnissen sind in den Druckschriften EP 2 594 493 A2, WO 2013/092735, EP 2 769 740 A1, EP 2 371 397, EP 2 724 731 und EP 2 769 922 A1 bekannt.

Zu diesem Zwecke sind aus dem Stand der Technik Abschirmeinrichtungen bekannt. Derzeit werden Abschirmungen von ionisierender Strahlung, wie z.B. Röntgenstrahlen, in Sterilisationsanlagen, beispielsweise durch sich drehende bzw. mitbewegte Strahlentrennelemente, bewerkstelligt, die zwischen den einzelnen zu behandelnden Objekten angeordnet sind oder die durch tunnelartige Anordnung von meist stehenden Trennelementen, deren Dimensionierung vom Durchmesser in Bezug auf den vorhandenen Bogenwinkel oder von der Spaltbreite abhängt. Der Nachteil derartiger mitbewegender bzw. mitrotierender Trennelemente besteht darin, dass die Lüftungstechnik bei unterschiedlichen Geschwindigkeiten in unterschiedlicher Weise beeinflusst wird. Weiterhin kann es bei rotierenden Trennelementen auch zu Freilegungen zwischen Ausleitflächen oder Übergabeflächen kommen und damit zu ungewollten Lecks. Auch ist bei derartigen Ausgestaltungen eine Strahlenabdichtung von Schleusensternen und unmittelbar an einer Strahlenquelle des Behandlungssterns schwierig. Problematisch sind derartige Ausgestaltungen auch bei engen Teilungen zwischen den einzelnen zu sterilisierenden Objekten. Daneben ist auch die Montage aufwendig und es kann auch zu einem Verhaken von verlorenen Behältnissen kommen. Das Vorsehen eines Strahlentunnels führt dazu, dass zum Teil relativ lange Wege nötig sind, um die Strahlung sicher abzuschirmen. Es hat sich hierbei gezeigt, dass Röntgenstrahlung erst dann für den Menschen ungefährlich wird, wenn sie vorher wenigstens dreimal abgelenkt bzw. umgelenkt wurde.

Der vorliegenden Erfindung gemäß Anspruch 1 liegt daher die Aufgabe zugrunde, eine Abschirmung gegenüber ionisierender Strahlung wie insbesondere Röntgenstrahlung bei derartigen Vorrichtungen zu verbessern. Daneben soll jedoch auch der Aufwand für derartige Abschirmungen gering gehalten werden. Diese Aufgaben werden erfindungsgemäß durch die Gegenstände der unabhängigen Ansprüche erreicht. Vorteilhafte Ausführungsformen und Weiterbildungen sind Gegenstand der Unteransprüche. Bei ionisierender Strahlung handelt es sich um Elektronenstrahlung, UV-Strahlung oder insbesondere Röntgenstrahlung handeln.

Eine erfindungsgemäße Vorrichtung zum Sterilisieren von Behältnissen weist eine Transporteinrichtung auf, welche die Behältnisse entlang eines vorgegebenen Transportpfades transportiert. Weiterhin weist die Vorrichtung wenigstens eine Beaufschlagungseinrichtung auf, welche die Behältnisse zum Zweck ihrer Sterilisation mit einer Strahlung, insbesondere einer elektromagnetischen Strahlung, beaufschlagt. Weiterhin weist die Vorrichtung eine Abschirmeinrichtung auf, welche im Rahmen der Sterilisation auftretende ionisierende Strahlung, insbesondere Röntgenstrahlung (gegenüber der Umgebung) abschirmt.

Erfindungsgemäß weist die Abschirmeinrichtung wenigstens eine den Transportpfad oder einen Transportbereich bzw. Transportraum der Behältnisse begrenzende Außenwandung auf, welche sich zumindest auch entlang des Transportpfades erstreckt, wobei diese Außenwandung wenigstens eine Ausnehmung aufweist, welche einen Hohlraum ausbildet, der dem Transportpfad der Behältnisse zugewandt ist, derart, dass bei der Sterilisation auftretende Röntgenstrahlung in diesen Hohlraum eintreten kann bzw. leitbar ist.

Erfindungsgemäß ist eine geometrische Struktur der Ausnehmung derart gestaltet, dass die Röntgenstrahlung wenigstens teilweise an Wandungen, welche die Ausnehmungen begrenzen, mehrfach reflektiert wird. Daneben könnten jedoch auch, wie oben erwähnt, auch innerhalb der Ausnehmung weitere derartige Wandungen bzw. Reflektionskörper vorgesehen sein.

Es wird daher weiter erfindungsgemäß vorgeschlagen, an einer begrenzenden Außenwandung eines Transportpfades bzw. Transportbereiches Ausnehmungen, im Folgenden auch als Strahlenfallen auszubilden, welche bewirken, dass in diesen die auftretende Röntgenstrahlung zumindest einmal, bevorzugt mehrmals reflektiert wird, sodass die Strahlung hierdurch bereits entscheidend abgeschwächt ist, sodass insgesamt die Abschirmung kleiner dimensioniert werden kann. Auf diese Weise kann auch der Bogenwinkel, der zur Abschirmung erforderlich ist, reduziert werden. Es wird daher vorgeschlagen, um eine derartige Verkürzung und somit eine Reduzierung des Bogenwinkels zu erhalten, so dass insbesondere an der Außenseite Strahlenfallen eingesetzt werden. Diese Strahlenfallen arbeiten dabei derart, dass ein Röntgenstrahl, der einen maximalen Weg in einen Behandlungstunnel zurücklegen könnte, nicht an der Außenwand des Tunnels zum Reflektieren gebracht wird, sondern in eine Öffnung der Außenabschirmung geleitet wird. Dies bedeutet, dass bevorzugt die Behältnisse innerhalb eines Tunnels transportiert werden.

Von dieser Vertiefung bzw. dem Hohlraum in der Außenwand kann der Strahl nicht wieder direkt in den Tunnel hinein strahlen, sondern benötigt hierzu wenigstens eine weitere Umlenkung. Dabei ist bevorzugt die Freilegung bzw. Ausnehmung etwas breiter in Richtung einer Außenkammer, um sämtliche auftretende Strahlung zuverlässig einzusammeln. Damit ist es, wie unten genauer gezeigt wird, denkbar, dass sich die Ausnehmung ausgehend von der Außenwand beispielsweise nach außen hin erweitert. Auch die Tiefe dieser Ausnehmung bzw. des Hohlraums mit der vorteilhaft dahinterliegenden Abschirmung wird vorteilhaft so gewählt, dass auch die auf eine Hinterwand der Ausnehmung auftreffenden Strahlen nur mit wenigstens einer weiteren Umlenkung wieder zurück in den Tunnel strahlen können.

Eine Winkelabmessung dieser Ausnehmung bzw. Strahlenfalle kann direkt von dem vollständigen Winkel des gesamten Tunnels abgezogen werden. Diese Ausnehmung kann dabei, wie unten genauer beschrieben wird, auch an einer zweiten Umlenkung eingesetzt werden.

Durch die erfindungsgemäße Ausnehmung lassen sich Winkelreduzierungen von 25° und mehr hinsichtlich des Tunnels erzielen. Es wäre jedoch auch möglich, dass in einem Decken- oder Bodenbereich des Pfades bzw. der Wandungen, die den Transportpfad begrenzen, verhindert wird, dass dort Röntgenstrahlung wieder zurück in den Tunnel reflektiert wird. Vorteilhaft können also auch in einem Bereich eines begrenzenden Bodens bzw. des begrenzenden Deckels entsprechende Ausnehmungen vorgesehen sein, die bevorzugt in etwa in der gleichen Größe ausgestaltet sind, wie die an einer hinteren Wand.

Ein Fehlen derartiger Ausnehmungen wäre zwar denkbar, es könnte jedoch vorkommen, dass reflektierende Strahlung die Funktion der Ausnehmung bzw. Strahlenfalle zumindest zu einem Teil aushebelt, was bedeuten würde, dass beispielsweise an einem Auslassfenster Röntgenstrahlung austreten würde bzw. könnte.

Durch die erfindungsgemäße Vorrichtung wird eine Verkürzung des benötigten Bogenwinkels zum Abschirmen der Röntgenstrahlen erreicht und damit kann auch eine Durchmesserreduktion des benötigten Sterndurchmessers erreicht werden, was wiederum eine Verringerung der Gehäusegröße bedeutet. Daneben kann eine wirksame Strahlenabdichtung geschaffen werden, die mit vorhandener Technik eine Strahlenabdichtung bewerkstelligt, die direkt im Anschluss eines Strahlenbehandlungskarussells angeordnet ist. Dabei werden keinerlei zusätzliche Bewegungen in einem Transferstern nötig, um die Strahlendichtigkeit zu gewährleisten. Auf diese Weise kann auch auf das Vorhandensein von rotierenden Trennelementen verzichtet werden, wodurch die Vorrichtung insgesamt einfacher gestaltet wird.

Es ist dabei möglich, dass die Ausnehmung vollständig als Hohlraum ausgebildet ist. Es wäre jedoch auch möglich, dass innerhalb des Hohlraums weitere Strahlungsumlenkelemente vorgesehen sind, die ebenfalls eine Mehrfachreflexion der Röntgenstrahlung innerhalb des Hohlraums ermöglichen. Auf diese Weise könnte erreicht werden, dass innerhalb des Hohlraums die Röntgenstrahlung wenigstens zweimal bzw. sogar wenigstens dreimal reflektiert wird.

Bei einer weiteren vorteilhaften Ausführungsform weist die Transporteinrichtung einen drehbaren Träger auf, an dem eine Vielzahl von Halteeinrichtungen zum Halten der Behältnisse angeordnet ist. Damit werden vorteilhaft die Behältnisse, insbesondere auch während ihrer Sterilisation, entlang eines kreisförmigen Transportpfades gefördert bzw. transportiert.

Bei einer weiteren vorteilhaften Ausführungsform weist die Beaufschlagungseinrichtung eine Elektronenstrahleinrichtung auf, welche wenigstens einen Wandungsbereich der Behältnisse mit Elektronen beaufschlagt. Dabei ist es möglich, dass diese Elektronenstrahleinrichtung die Behältnisse von außen zu deren Sterilisation beaufschlagt, es wäre jedoch auch denkbar, dass die Beaufschlagungseinrichtung einen stangenartigen Körper aufweist, der durch die Mündung der Behältnisse in dieselben einführbar ist. Daneben könnten auch mehrere unterschiedliche Gattungen an Beaufschlagungseinrichtungen vorgesehen sein. Dabei ist es möglich und bevorzugt, dass die Beaufschlagungseinrichtung stationär angeordnet ist und sich die Behältnisse dieser gegenüber bewegen. Es wäre jedoch auch denkbar, dass die Beaufschlagungseinrichtung selbst beweglich und insbesondere beweglich mit den Behältnissen angeordnet ist.

Bei einer weiteren vorteilhaften Ausführungsform weist die Vorrichtung mehrere Ausnehmungen auf, die entlang des Transportpfades der Behältnisse hintereinander angeordnet sind. Auf diese Weise kann der Umfangswinkel, der für eine Abschwächung nötig ist, noch weiter reduziert werden. Dabei wäre es auch möglich, dass die Ausnehmungen derart gestaltet sind, dass beispielsweise die Außenwandung eine im Wesentlichen wellenförmige Struktur aufweist. Daneben können, wie oben erwähnt, sowohl in der besagten Außenwandung als auch in einer Deckel- oder Bodenwandung derartige Ausnehmungen angeordnet sein. Es wird dabei darauf hingewiesen, dass auch eine Deckel- und eine Bodenwandung entsprechende Außenwandungen darstellen. Damit werden diese Deckel- und Bodenwandung im Folgenden auch genauer als Deckelaußenwandung bzw. Bodenaußenwandung bezeichnet.

Bei der Innenwandung hingegen handelt es sich um diejenige Wandung, welche bezüglich des Transportpfades der Behältnisse radial innen angeordnet ist und auf diese Weise den Transporttunnel der Behältnisse begrenzt. Auch an der den Kanal begrenzenden Innenwandung können die hier beschriebenen Ausnehmungen vorgesehen sein.

Bei einer weiteren vorteilhaften Ausführungsform weist daher die Vorrichtung auch eine den Transportpfad der Behältnisse bzw. den Transportbereich der Behältnisse begrenzende Innenwandung auf, die sich bevorzugt ebenfalls in Richtung des Transportpfades erstreckt. Auf diese Weise wird ein Transportkanal bzw. Transporttunnel ausgebildet.

Bei einer weiteren vorteilhaften Ausführung werden die Strahlungsfallen in Bezug auf die transportierten Behältereinheiten beweglich ausgeführt. Erfolgt an einem Behälter eine verstärkte Reflexion von Störstrahlung, dann wird diese in der Strahlenfalle aufgenommen. Insbesondere ist hier die Gegenstromführung von Behältereinheit und Strahlungsfalle zu berücksichtigen. Um den notwendigen Abschirmweg weiter zu reduzieren, kann punktuell die Strahlenfalle so auf die reflektierende Behältereinheit getaktet werden, dass an einem Messpunkt die periodisch auftretende Dosis abgefangen wird. Daher wäre es auch denkbar, dass die Strahlungsfallen selbst beweglich angeordnet sind. Dabei ist sowohl eine Bewegung der Strahlenfallen in einer Transportrichtung (oder entgegengesetzt zu dieser Transportrichtung) der Behältnisse denkbar (mit der gleichen oder einer anderen Geschwindigkeit) als auch eine Bewegung in hiervon abweichenden Richtungen. So könnte sich beispielsweise auch eine Wandung, an der die Strahlungsfallen angeordnet sind, bewegen.

Bei einer weiteren vorteilhaften Ausführungsform bildet die Vorrichtung einen Reinraum aus, innerhalb dessen die Behältnisse während ihrer Sterilisation gefördert werden. Dabei ist es möglich, dass innerhalb dieses Reinraums ein Überdruck herrscht, sodass keine Verunreinigungen von außen in den Reinraum eindringen können. Weiterhin wäre es möglich, dass eine Dichtungseinrichtung vorgesehen ist, welche diesen Reinraum gegenüber der Umgebung abdichtet. Dabei ist es denkbar, dass diese Dichtungseinrichtung zwei bezüglich einander bewegliche Elemente bzw. Wandungsabschnitte aufweist.

Es wäre jedoch auch möglich, dass die Behältnisse nicht innerhalb eines Reinraums sterilisiert werden, sondern beispielsweise noch vor Erreichen eines Reinraums. Auf diese Weise kann beispielsweise eine Vorsterilisierung ermöglicht werden, welche eine spätere Hauptsterilisierung bzw. Nachsterilisierung hinsichtlich deren Aufwand reduzieren kann.

Die vorliegende Erfindung ist weiterhin auf ein Verfahren zum Behandeln von Behältnissen gerichtet. Dabei werden die Behältnisse mittels einer Transporteinrichtung entlang eines vorgegebenen Transportpfades transportiert und die Behältnisse werden mittels wenigstens einer Beaufschlagungseinrichtung zum Zwecke ihrer Sterilisation mit einer elektromagnetischen Strahlung beaufschlagt, wobei während dieser Beaufschlagung Röntgenstrahlung entsteht und wobei diese entstehende Röntgenstrahlung wenigstens teilweise mittels einer Abschirmeinrichtung abgeschirmt wird. Erfindungsgemäß gelangt die Röntgenstrahlung wenigstens teilweise in eine Ausnehmung, welche an einer den Transportpfad der Behältnisse begrenzenden Wandung, insbesondere einer Außenwandung, angeordnet ist.

Damit wird auch verfahrensseitig vorgeschlagen, dass eine Ausnehmung in der Wandung gebildet ist, welche zum Abschwächen der Röntgenstrahlung, insbesondere durch wenigstens einfache und bevorzugt mehrfache Reflexion dieser Röntgenstrahlung, innerhalb der Ausnehmung dient.

Bei einer bevorzugten Variante werden die Behältnisse entlang eines kreisförmigen Transportpfades transportiert.

Weitere Vorteile und Ausführungsformen ergeben sich aus den beigefügten Zeichnungen. Darin zeigen:
- Fig. 1: Eine schematische Darstellung einer Vorrichtung zum Sterilisieren von Behältnissen nach dem internen Stand der Technik der Anmelderin;
- Fig. 2: eine schematische Darstellung zur Veranschaulichung des Verlaufes von Röntgenstrahlen bei der Vorrichtung nach Fig. 1;
- Fig. 3: eine schematische Darstellung einer erfindungsgemäßen Vorrichtung;
- Fig. 4: eine weitere Ausführungsform der erfindungsgemäßen Vorrichtung;
- Fig. 5: einen Strahlenverlauf bei der Vorrichtung aus Fig. 4; und
- Fig. 6: eine weitere Ausführungsform der vorliegenden Erfindung.

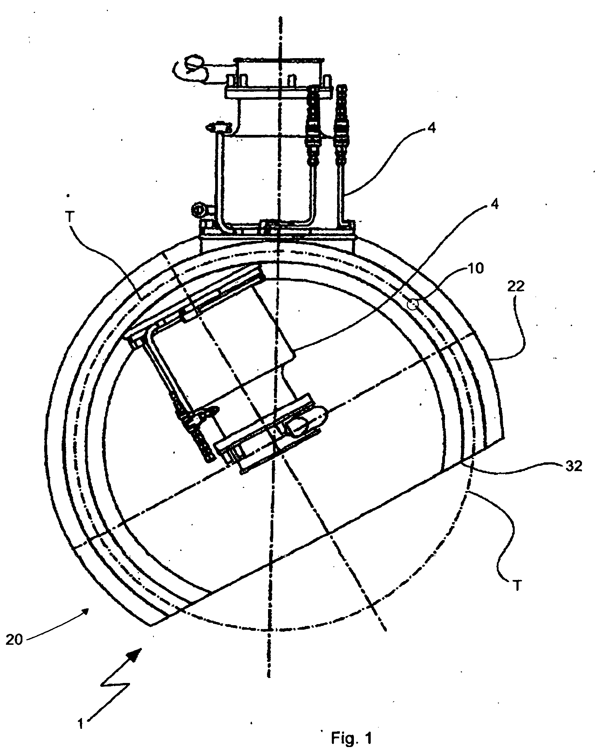

Fig. 1 zeigt eine grob schematische Darstellung einer Vorrichtung 1 zum Sterilisieren von Behältnissen nach dem internen Stand der Technik der Anmelderin. Dabei werden Behältnisse 10 (nur schematisch gezeigt) zwischen zwei Wandungen 22 und 32 entlang eines hier kreisförmigen Transportpfades T transportiert. Das Bezugszeichen 1 bezieht sich auf die Vorrichtung in ihrer Gesamtheit. Diese Vorrichtung weist hier zwei Beaufschlagungseinrichtungen bzw. Strahlungseinrichtungen 4 auf, welche eine Außenwandung der Behältnisse mit Strahlung, beispielsweise mit Elektronenstrahlung, beaufschlagt. Zwischen den beiden Wandungen 22 und 32 und eine (nicht gezeigte) Bodenwandung wird ein Kanal gebildet, innerhalb dessen die Kunststoffvorformlinge 10 gefördert werden.

Die Vorrichtung 1 weist eine (nicht gezeigte) Transporteinrichtung auf, welche die Behältnisse transportiert. Dabei wäre es denkbar, dass ein drehbarer Träger vorgesehen ist, an dem Greifelemente und/oder Haltedorne zum Greifen bzw. Halten der Behältnisse 10, bei denen es sich insbesondere um Kunststoffvorformlinge handelt, angeordnet sind. Auch wäre es denkbar, dass auch die Innenwandung 32 drehbar angeordnet ist an dieser Innenwandung die Greifelemente bzw. Haltedorne angeordnet sind. Das Bezugszeichen 20 bezieht sich in seiner Gesamtheit auf die Abschirmeinrichtung, welche die entstehende Röntgenstrahlung abschirmt, wobei beispielsweise die Außenwandung eine Komponente dieser Abschirmeinrichtung 20 ist.

Die gezeigte Vorrichtung 1 ist bevorzugt innerhalb eines Moduls angeordnet, das weitere Behandlungsstationen, insbesondere eine weitere Behandlungsstation aufweist. Die Kunststoffvorformlinge durchlaufen das Modul bevorzugt derart, dass diese nach einem Einlaufstern von der gezeigten Vorrichtung 1 auf deren Außenseite mittels Ladungsträgerstrahlung, insbesondere mittels Elektronenstrahlen (in Betracht kämen jedoch auch etwa Protonen oder alpha-Teilchen) sterilisiert werden. Während der Außenbestrahlung werden die Kunststoffvorformlinge bevorzugt mittels eines Innengreifers an deren Innenfläche gehaltert und durch einen tunnelartigen Kanal transportiert. Im Anschluss daran werden die Kunststoffvorformlinge bevorzugt mittels eines aseptischen Transportsterns bzw. Teilungsverzugssterns zu einer nachfolgenden Behandlungsvorrichtung (nicht gezeigt) geführt, welche die Kunststoffvorformlinge (ebenfalls bevorzugt mittels Elektronenstrahlung) auf deren Innenseite entkeimt.

Dabei werden die Kunststoffvorformlinge auf deren Außenseite gehaltert, während ein Elektronenstrahlemitter wenigstens teilweise in den Vorformling eingeführt wird. Bei dieser Innenentkeimung findet eine Relativbewegung zwischen dem Emitter und dem Behältnis bzw. dem Vorformling statt. Im Anschluss an die Innenentkeimung werden die Vorformlinge mittels eines weiteren Transportsterns aus dem Modul heraus befördert. Die genannten Vorrichtungen innerhalb des Moduls sind dabei bevorzugt in ihrer Gesamtheit eingehaust, damit keine Elektronen- bzw. Röntgenstrahlung austreten kann. Insbesondere ist es daher auch möglich, dass innerhalb dieser Modul-Einhausung Strahlenfallen angeordnet werden, die das häufige Ablenken der Röntgenstrahlung innerhalb des Moduls gewährleisten, um das Modul kompakt halten zu können.

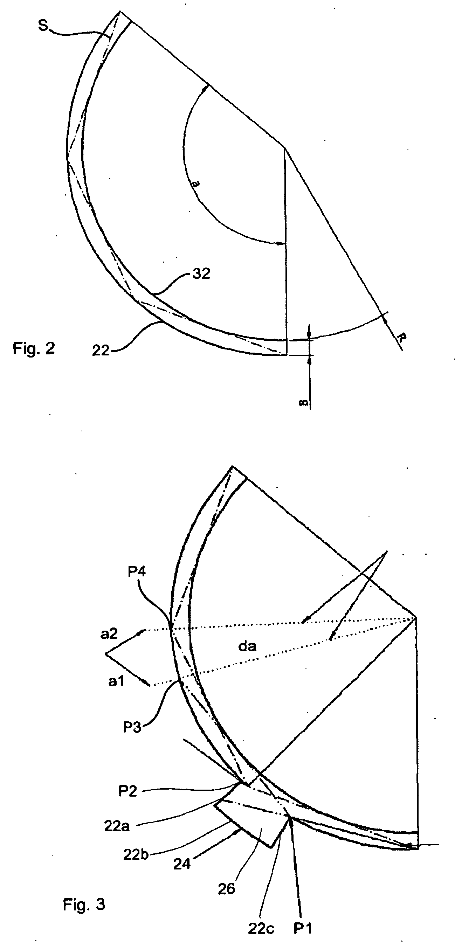

Fig. 2 zeigt eine Darstellung zur Veranschaulichung eines Strahlenverlaufs bei der in Fig. 1 gezeigten Vorrichtung. Dabei bezieht sich das Bezugszeichen S auf entstehende Röntgenstrahlung, die hier mehrfach reflektiert und auf diese Weise abgeschwächt wird. Das Bezugszeichen a kennzeichnet einen Bogen, der demjenigen Umfangswinkel entspricht, der nötig ist, um eine ausreichende Abschwächung der Röntgenstrahlung zu erreichen. Diese ergibt sich aus dem Winkel, der zwischen drei Punkten liegt, an denen die Strahlung dreimal an der Außenwandung reflektiert wird. Das Bezugszeichen B kennzeichnet den Abstand zwischen den beiden Wandungen 22 und 32 und damit die Breite des Spalts, innerhalb dessen die Kunststoffvorformlinge 10 transportiert werden. Das Bezugszeichen R kennzeichnet einen Krümmungsradius der Innenwandung 32. Man erkennt, dass beide Wandungen hier kreisförmig gekrümmt sind und bevorzugt jeweils als Mittelpunkt den Mittelpunkt des Transportpfads der Kunststoffvorformlinge aufweisen.

Fig. 3 zeigt eine schematische Darstellung der erfindungsgemäßen Vorrichtung. Bei dieser Ausgestaltung ist an der Außenwandung 22 eine Ausnehmung 24 bzw. eine Strahlenfalle 24 angeordnet. Diese Ausnehmung bildet einen Hohlraum 26 aus, in den die Röntgenstrahlen S einlaufen können. Weiterhin weist diese Ausnehmung 24 einen sich radial nach außen erweiternden Querschnitt auf. Durch diesen Querschnitt kann die Effizienz der Ausnehmung bzw. der Strahlenfalle 24 weiter verbessert werden. Die Bezugszeichen 22a, 22b, 22c beziehen sich auf Wandungen, welche den Hohlraum begrenzen und an denen die entstehende Röntgenstrahlung ebenfalls reflektiert werden kann. Auch wäre es in Abhängigkeit von den Abmessungen der Ausnehmung auch denkbar, dass entstehende Röntgenstrahlung hintereinander an mehreren dieser Wandungen 22a, 22b, 22c reflektiert wird. Das Bezugszeichen da kennzeichnet wieder die Winkeldifferenz, die sich aus den beiden Winkel a2 und a1 ergibt und die die Reduzierung veranschaulicht, die durch die Einfügung der Strahlenfalle 24 erreicht wird.

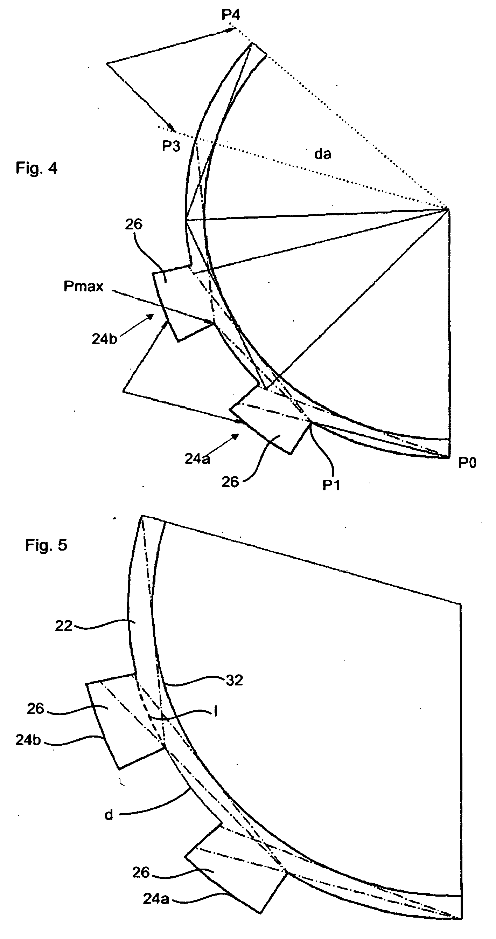

Fig. 4 zeigt eine weitere Ausführungsform der erfindungsgemäßen Vorrichtung. Bei dieser Ausführungsform sind an bzw. in der Außenwandung zwei Ausnehmungen 24a, 24b angeordnet, welche jeweils die Hohlräume 26 ausbilden. Das Bezugszeichen da kennzeichnet den Winkel bzw. die Umfangsstrecke, welche durch das Vorhandensein dieser beiden Ausnehmungen bzw. Strahlenfallen eingespart werden kann. Dabei bezieht sich das Bezugszeichen P4 auf den Punkt, in dem die Strahlen ausgehend von dem Punkt P0 (Strahleneintritt) zum dritten Mal reflektiert werden. Das Bezugszeichen P3 kennzeichnet den Punkt, den die Strahlen bei Vorhandensein der beiden Strahlenfallen maximal erreichen können, wobei mit dem Bezugszeichen Pmax jeweils der Punkt Pmax der jeweilige maximale Reflexionspunkt mit Strahlenfalle gekennzeichnet ist.

Fig. 5 zeigt einen Strahlenverlauf bei der Vorrichtung aus Fig. 4, wobei hier nur die Strahlenverläufe eingezeichnet sind, die sich maximal bei Vorhandensein der beiden Ausnehmungen ergeben können. Vorteilhaft sind die Ausnehmungen 24a, 24b in einer Umfangsrichtung der Vorrichtung 1 in einem vorgegebenen Abstand zueinander angeordnet. Dabei kann auch dieser Abstand in Abhängigkeit von einer Umfangsgröße der jeweiligen Ausnehmung derart gewählt werden, dass ein maximaler Strahlungsweg der entstehenden Röntgenstrahlung bestmöglich reduziert wird. Bevorzugt liegt ein Verhältnis zwischen einer Länge I der beiden Ausnehmungen 24a, 24b in Umfangsrichtung und einem Abstand d zwischen diesen benachbarten Ausnehmungen zwischen 1:2 und 2:1, bevorzugt zwischen 1:1,5 und 1,5:1 und besonders bevorzugt zwischen 1:1,2 und 1,2 : 1.

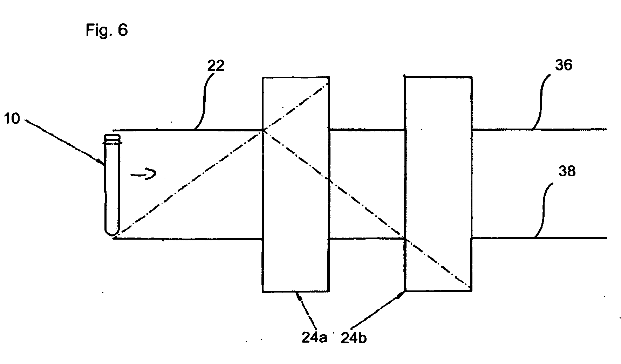

Fig. 6 zeigt eine Seitenansicht einer weiteren Ausführungsform der erfindungsgemäßen Vorrichtung. Man erkennt hier, dass auch in einer Deckenwandung 36 und einer Bodenwandung 38 entsprechende Ausnehmungen vorhanden sind. So wäre es denkbar, dass die oben beschriebenen Ausnehmungen 24 bzw. 24a, 24b derart gestaltet sind, dass sie sich auch in den Bereich der Deckenwandung und/oder der Bodenwandung erstrecken. Man erkennt, dass auch durch diese Maßnahmen eine Verkürzung des maximal von der Strahlung zurückzulegenden Weges bis hin zu der dritten Reflexion erreicht werden kann.

Vorteilhaft sind die Wandungen wenigstens abschnittsweise und bevorzugt vollständig aus einem Röntgenstrahlen abschirmenden Material wie beispielsweise Blei hergestellt.

Es wäre weiterhin auch denkbar und bevorzugt, dass die in Fig. 1 gezeigten Strahlungseinrichtungen 4 derart angeordnet sind, dass Strahlung jedenfalls nicht bis zu einer Ausgangsschleuse und/oder einer Eingangsschleuse der Vorrichtung gelangen kann.

### Bezugszeichenliste

- 1: Vorrichtung in ihrer Gesamtheit
- 4: Strahlungseinrichtungen
- 10: Behältnisse
- 20: Abschirmeinrichtung
- 22,32: Wandungen
- 22a, 22b, 22c: Wandungen
- 24: Ausnehmung / Strahlenfalle
- 24a, 24b: Ausnehmungen
- 26: Hohlraum
- 32: Innenwandung
- 36: Deckenwandung
- 38: Bodenwandung
- T: kreisförmiger Transportpfad
- S: Röntgenstrahlung
- B: Breite des Spalts
- R: Radius der Innenwandung 32
- a: Bogen
- a1,a2: Winkel
- da: Winkeldifferenz
- P0 - P5: Punkte
- I: Länge der Ausnehmung in Umfangsrichtung
- d: Abstand zwischen zwei Ausnehmungen in Umfangsrichtung

## Patentansprüche

1. Vorrichtung (1) zum Sterilisieren von Behältnissen (10) mit einer Transporteinrichtung, welche die Behältnisse (10) entlang eines vorgegebenen Transportpfads transportiert, mit wenigstens einer Beaufschlagungseinrichtung (4), welche die Behältnisse (10) zum Zweck ihrer Sterilisation mit einer Elektronenstrahlung, UV-Strahlung oder Röntgenstrahlung beaufschlagt und mit einer Abschirmeinrichtung (20), welche im Rahmen der Sterilisation auftretende ionisierende Strahlung abschirmt,
**dadurch gekennzeichnet, dass**
die Abschirmeinrichtung (20) wenigstens eine den Transportpfad der Behältnisse (10) begrenzende Außenwandung (22) aufweist, welche sich entlang des Transportpfads erstreckt die Vorrichtung eine den Transportpfad der Behältnisse begrenzende Innenwandung (32) aufweist, die sich bevorzugt ebenfalls in Richtung des Transportpfades erstreckt, wobei die Außenwandung wenigstens eine Ausnehmung (24) aufweist, welche einen Hohlraum (26) ausbildet, der dem Transportpfad der Behältnisse (10) zugewandt ist, derart, dass bei der Sterilisation auftretende ionisierende Strahlung in diesen Hohlraum eintreten kann, wobei die geometrische Struktur der Ausnehmung (24) derart gestaltet ist, dass die ionisierende Strahlung wenigstens teilweise an Wandungen (22a, 22b, 22c), welche die Ausnehmung (24) begrenzen, mehrfach reflektiert wird, und wobei die Ausnehmung (24) in Form einer Strahlenfalle ausgebildet ist, wobei die Ausnehmung dabei weiter derart ausgebildet ist, dass ein Strahl nicht an der Außenwandung (24) zum Reflektieren gebracht wird, sondern in die Ausnehmung (24) geleitet wird, und von dieser Ausnehmung (24) der Strahl nicht wieder direkt in den Tunnel hinein strahlen kann, sondern hierzu wenigstens eine weitere Umlenkung benötigt.

2. Vorrichtung (1) nach Anspruch 1
**dadurch gekennzeichnet, dass**
die Transporteinrichtung (2) einen drehbaren Träger aufweist, an dem eine Vielzahl von Halteeinrichtungen zum Halten der Behältnisse (10) angeordnet ist.

3. Vorrichtung (1) nach wenigstens einem der vorangegangenen Ansprüche,
**dadurch gekennzeichnet, dass**
die Beaufschlagungseinrichtung (4) eine Elektronenstrahleinrichtung aufweist, welche wenigstens einen Wandungsbereich der Behältnisse mit Elektronen beaufschlagt.

4. Vorrichtung (1) nach wenigstens einem der vorangegangenen Ansprüche,
**dadurch gekennzeichnet, dass**
die Vorrichtung (1) mehrere Ausnehmungen (24a,b) aufweist, die entlang des Transportpfads der Behältnisse (10) hintereinander angeordnet sind.

5. Vorrichtung (1) nach wenigstens einem der vorangegangenen Ansprüche,
**dadurch gekennzeichnet, dass**
die Vorrichtung (1) einen Reinraum ausbildet, innerhalb dessen die Behältnisse (10) während ihrer Sterilisation gefördert werden.

6. Verfahren zum Behandeln von Behältnissen (10) in einer Vorrichtung gemäß Anspruch 1, wobei die Behältnisse (10) mittels einer Transporteinrichtung entlang eines vorgegebenen Transportpfades transportiert werden und wobei die Behältnisse (10) mittels wenigstens einer Beaufschlagungseinrichtung (4) zum Zwecke ihrer Sterilisation mit einer Elektronenstrahlung, UV-Strahlung oder Röntgenstrahlung beaufschlagt werden, wobei während dieser Beaufschlagung ionisierende Strahlung entsteht, und wobei diese entstehende ionisierende Strahlung mittels einer Abschirmeinrichtung (20) abgeschirmt wird, **dadurch gekennzeichnet, dass**
die ionisierende Strahlung wenigstens teilweise in eine Ausnehmung (24) gelangt,
welche an einer den Transportpfad der Behältnisse (10) begrenzenden Wandung (22) angeordnet ist, wobei die geometrische Struktur der Ausnehmung (24) derart gestaltet ist, dass die ionisierende Strahlung wenigstens teilweise an Wandungen (22a, 22b, 22c), welche die Ausnehmung (24) begrenzen, mehrfach reflektiert wird, und wobei die Ausnehmung (24) in Form einer Strahlenfalle ausgebildet ist, wobei die Ausnehmung dabei weiter derart ausgebildet ist, dass ein Strahl nicht an der Außenwandung (24) zum Reflektieren gebracht wird, sondern in die Ausnehmung (24) geleitet wird, und von dieser Ausnehmung (24) der Strahl nicht wieder direkt in den Tunnel hinein strahlen kann, sondern hierzu wenigstens eine weitere Umlenkung benötigt.

7. Verfahren nach Anspruch 6,
**dadurch gekennzeichnet, dass**
die Behältnisse entlang eines kreisförmigen Transportpfades transportiert werden.

## Claims

1. An apparatus (1) for the sterilization of containers (10) with a transport device which transports the containers (10) along a pre-defined transport path, with at least one actuating device (4) which acts upon the containers (10) with an electromagnetic radiation, UV radiation or X-radiation, for the purpose of the sterilization thereof, and with a screening device (20) which screens off ionizing radiation which is formed in the context of the sterilization, **characterized in that** the screening device (20) has at least one outer wall (22) which limits the transport path of the containers (10) and which extends along the transport path, the apparatus comprising an inner wall (32) which limits the transport path of the containers and also extends along the transport path, wherein the outer wall comprises at least one recess (24) which forms a cavity (26) which faces the transport path of the containers (10) in such a way that ionizing radiation occurring during the sterilization can enter this cavity wherein the geometrical structure of the recess (24) is designed in such a way that the ionizing radiation is reflected several times at least in part on walls (22a, 22b, 22c) which limit the recess (24) and wherein the recess (24) is designed in the form of a beam trap, the recess in this respect being further designed in such a way that a reflection of a beam of not intended at the outer wall (24) but said beam is introduced in the recess (24) and from this recess (24) the beam cannot directly penetrate the tunnel again but for this purpose at least a further deviation is necessary.

2. An apparatus (1) according to claim 1, **characterized in that** the transport device (2) has a rotatable carrier on which a plurality of holding devices for holding the containers (10) are arranged.

3. An apparatus (1) according to at least one of the preceding claims, **characterized in that** the actuating device (4) has an electron radiation device which acts upon at least one wall region of the containers with electrons.

4. An apparatus (1) according to at least one of the preceding claims, **characterized in that** the apparatus (1) comprises several recesses (24a, b) which are arranged in succession along the transport path of the containers (10).

5. An apparatus (1) according to at least one of the preceding claims, **characterized in that** the apparatus (1) forms a clean room inside which the containers (10) are transported during the sterilization thereof.

6. A method of treating containers (10) in an apparatus according to claim 1, wherein the containers (10) are transported along a pre-defined transport path by means of a transport device and wherein the containers (10) are acted upon with an electromagnetic radiation, UV radiation or X-radiation by means of at least one actuating device (4) for the purpose of their sterilization, wherein ionizing radiation occurs during this actuating process and wherein this ionizing radiation which occurs is screened off by means of a screening device (20), **characterized in that** the ionizing radiation arrives at least in part in a recess (24) which is arranged in a wall (22) limiting the transport path of the containers (10), wherein the geometrical structure of the recess (24) is designed in such a way that the ionizing radiation is reflected several times at least in part on walls (22a, 22b, 22c) which limit the recess (24) and wherein the recess (24) is designed in the form of a beam trap, the recess in this respect being further designed in such a way that a reflection of a beam is not intended at the outer wall (24) but said beam is introduced in the recess (24) and from this recess (24) the beam cannot directly penetrate the tunnel again but for this purpose at least a further deviation is necessary.

7. A method according to claim 6, **characterized in that** the containers are transported along a circular transport path.

## Revendications

1. Dispositif (1) de stérilisation de contenants (10) comprenant un système de transport, lequel transporte les contenants (10) le long d'un chemin de transport prédéfini, au moins un système d'exposition (4), lequel, pour stériliser les contenants (10), les expose à un rayonnement par faisceau électronique, à un rayonnement UV ou à un rayonnement X, et un système de blindage (20), lequel fait écran au rayonnement ionisant se produisant dans le cadre de la stérilisation,
**caractérisé en ce que**
le système de blindage (20) comprend au moins une paroi extérieure (22) délimitant le chemin de transport des contenants (10), laquelle s'étend le long du chemin de transport, le dispositif comprenant une paroi intérieure (32) qui délimite le chemin de transport des contenants et qui s'étend de préférence également en direction du chemin de transport, la paroi extérieure présentant au moins un évidement (24), lequel forme une cavité (26) qui est tournée vers le chemin de transport des contenants (10), de telle manière que le rayonnement ionisant se produisant lors de la stérilisation peut pénétrer dans cette cavité, la structure géométrique de l'évidement (24) étant configurée de telle sorte que le rayonnement ionisant est réfléchi au moins en partie sur les parois (22a, 22b, 22c), lesquelles délimitent l'évidement (24), et l'évidement (24) prenant la forme d'un piège à rayons, l'évidement étant conçu en outre de telle sorte qu'un rayon n'est pas amené à être réfléchi sur la paroi extérieure (24), mais est guidé dans l'évidement (24), et de cet évidement (24), le rayon ne peut à nouveau pénétrer directement à l'intérieur du tunnel, mais au moins une autre déviation est nécessaire à cela.

2. Dispositif (1) selon la revendication 1,
**caractérisé en ce que**
le système de transport (2) comprend un support rotatif sur lequel sont disposés une pluralité de systèmes de retenue servant à retenir les contenants (10).

3. Dispositif (1) selon au moins l'une des revendications précédentes,
**caractérisé en ce que**
le système d'exposition (4) comprend un système de faisceau d'électrons, lequel expose au moins une zone de paroi des contenants à des électrons.

4. Dispositif (1) selon au moins l'une des revendications précédentes,
**caractérisé en ce que**
le dispositif (1) comprend plusieurs évidements (24a,b), qui sont disposés les uns derrière les autres le long du chemin de transport des contenants (10).

5. Dispositif (1) selon au moins l'une des revendications précédentes,
**caractérisé en ce que**
le dispositif (1) forme une salle blanche à l'intérieur de laquelle les contenants (10) sont transportés pendant leur stérilisation.

6. Procédé de traitement de contenants (10) dans un dispositif selon la revendication 1, les contenants (10) étant transportés au moyen d'un système de transport le long d'un chemin de transport prédéfini et les contenants (10), pour être stérilisés, étant exposés au moyen d'au moins un système d'exposition (4) à un rayonnement par faisceau d'électrons, à un rayonnement UV ou à un rayonnement X, un rayonnement ionisant se formant pendant cette exposition, et écran étant fait à ce rayonnement ionisant formé au moyen d'un système de blindage (20),
**caractérisé en ce que**
le rayonnement ionisant parvient au moins en partie dans un évidement (24), lequel est agencé sur une paroi (22) délimitant le chemin de transport des contenants (10), la structure géométrique de l'évidement (24) étant configurée de telle sorte que le rayonnement ionisant est réfléchi à plusieurs reprises au moins en partie sur les parois (22a, 22b, 22c), lesquelles délimitent l'évidement (24), et l'évidement (24) prenant la forme d'un piège à rayons, l'évidement étant conçu en outre de telle sorte qu'un rayon n'est pas amené à être réfléchi sur la paroi extérieure (24), mais est guidé dans l'évidement (24), et de cet évidement (24), le rayon ne peut pas à nouveau pénétrer directement à l'intérieur du tunnel, mais au moins une autre déviation est nécessaire à cela.

7. Procédé selon la revendication 6,
**caractérisé en ce que**
les contenants sont transportés le long d'un chemin de transport circulaire.
